# EUROPEAN PATENT APPLICATION

(11) **EP 2 275 527 A1**
(43) Date of publication of application: **19.01.2011**
(21) Application number: 09100362.4
(22) Date of filing: 17.07.2009
(51) Int. Cl.: C12N 1/20, C12R 1/225

(54) **Animal component-free culture medium for bacterial fermentation**

(71) Applicant: ActoGeniX NV, 9052 Zwijnaarde (BE)
(72) Inventor: The designation of the inventor has not yet been filed

(57) **Abstract**

The invention relates to a simple, high-yield animal-free culture media for lactic acid bacterial strains in general and a well-defined process for obtaining a high-yield of said bacteria making use of said medium. The invention further relates to the use of said medium for culturing recombinant lactic acid bacterial strains for recombinant protein isolation or therapeutic purposes.

## Description

### Field of the Invention

The invention relates generally to the field of animal component-free culture media for lactic acid bacterial strains and the process for obtaining a high yield of said bacteria making use of said medium.

### Background to the Invention

Lactic acid bacterial strains such as for example *Lactococcus, Lactobacillus* or *Bifidobacterium* species, are being used in the food industry for many years because they are able to convert sugars (including lactose) and other carbohydrates into lactic acid, and are accordingly useful in the preparation of fermented dairy foods such as yogurt and certain cheese varieties.

It has also been established that said lactic acid bacterial strains are beneficial to the body's naturally occurring gut flora, an ecology of microbes, and are accordingly recommended by physicians, and, more frequently, by nutritionists, after a course of antibiotics, or as part of the treatment for gut related candidiasis. In these indications, the bacteria that work well with our bodies may decrease in number, an event that allows harmful competitors to thrive, to the detriment of our health. Claims are made that living food grade bacteria strengthen the immune system to combat allergies, excessive alcohol intake, stress, exposure to toxic substances, and other diseases.

More recently, recombinant strains of said food grade bacteria are also being used as promising tools in the intestinal delivery of therapeutic molecules. For example, intestinal delivery of viable recombinant hIL-10 producing *Lactococcus lactis* (Thy12) was found to have a clinical effect in patients suffering from Crohn's disease (Steidler L. et al., Nat. Biotechnol. 21(7) (2003) 785-789), and in the treatment of Acquired Lactase Deficiency, viable lactase producing bacteria, such as *Lactobacillus acidophilus,* and *Lactobacillus bulgaricus* are used in a lactase replacement therapy (US 6,008,027).

In conventional culture media for lactic acid bacterial strains, proteinaceous material from animal origin are used such as for example in the M17 medium for Lactic Streptococci (DSMZ Medium 449), the Lactobacillus medium IV (DSMZ Medium 859), the lactobacilli MRS broth (BD Difco - 2288130), and the Lactobacillus selection broth (BD Difco -211331). These compounds are used in the conviction that some growth factors, essential for the growth of the bacteria, are only present in compounds of animal origin such as blood, meat etc. However, the use of proteinaceous material of animal origin in bacterial culture media gives rise to concerns about possible contamination of the media with for example the bovine spongiform encephalopathy (BSE) causative agent, animal viruses, other prions or other infectious and harmful agents. Therefore, governmental requirements restrict the use of bacteria cultivated in media containing proteinaceous material of animal origin, especially in therapeutic applications.

Accordingly, non-animal derived culture media have been developed in the past, however, these comprise often complex formulations or they have been specifically developed for particular micro-organism such as the streptococci, meningococci and *Haemophilus influenza* hereinafter.

WO94/09115 discloses a culture medium containing no complex animal components and is optimized for the production of Streptococcus pneumoniae and the isolation of pneumococcal capsular polysaccharide which can be used to prepare vaccines. This SYG medium contains Yeast Extract, Soy Peptone, Resazurin, Vitamin K, Hemin, Sodium Bicarbonate, L-cysteine HCl, Glucose, HEPES buffer (pH 7.3 -7.8) and a salt solution containing CaCl₂, MgSO₄, K₂HPO₄, KH₂PO₄, NaHCO₃ and NaCl.

US6933137 discloses an animal-free chemically defined culture medium specifically developed for meningococci comprising sodium phosphate, soya peptone, monosodium glutamate, potassium chloride, magnesium sulphate, L-cysteine and glucose and that does not contain NH₄Cl.

US2002/0137140 discloses a complex lactic acid bacteria culture medium comprising 19 amino acids, K₂SO₄, KH₂PO₄/K₂HPO₄, Na-acetate, CaCl₂, MgCl₂, FeSO₄, vitamins, micronutrients and citric acid.

EP0983342B1 discloses a medium comprising at least 20% of dry weight of a soy bean derived protein composition for cultivating *Haemophilus influenza.* The example medium for cultivating *Haemophilus influenza* comprises glutamic acid, Na₂HPO₄.12H₂O, KCl, NaCl, NH₄Cl, NaOH, soytone, proteose peptone, yeast extract, glucose NAD, Tris and Hemin.

Apart from lacking non-animal derived proteinaceous compounds, the accordingly optimized media should ideally only contain essential components, including a carbon source, an energy source, a nitrogen source and essential salts; be easily prepared in a reproducible manner; and support robust high-cell density cultures, with a preferably higher yield compared to the conventional media.

Given the shortcomings of the currently available culture media for lactic acid bacterial strains, it was an object of the present invention to provide an optimized, non-expensive, simple, high-yield animal component-free culture medium.

The medium disclosed in this invention overcomes most of the difficulties of the conventional bacterial culture medium in that it is much more simplified and as such also easier and cheaper for cultivating large amounts of bacteria. Furthermore, we have surprisingly found that eliminating soy peptone and sodium-containing compounds as well as using NH₄OH as a pH regulator results in a significantly higher yield at the end of the cultivating process.

### Brief Description of the Drawings

**Figure 1****.** Effect of removing meat extract from the M17 medium on growth of *Lactococcus Lactis* as determined by measuring OD₆₀₀ values over time at 2 different concentrations of glucose in the medium (0,5% and 5%).

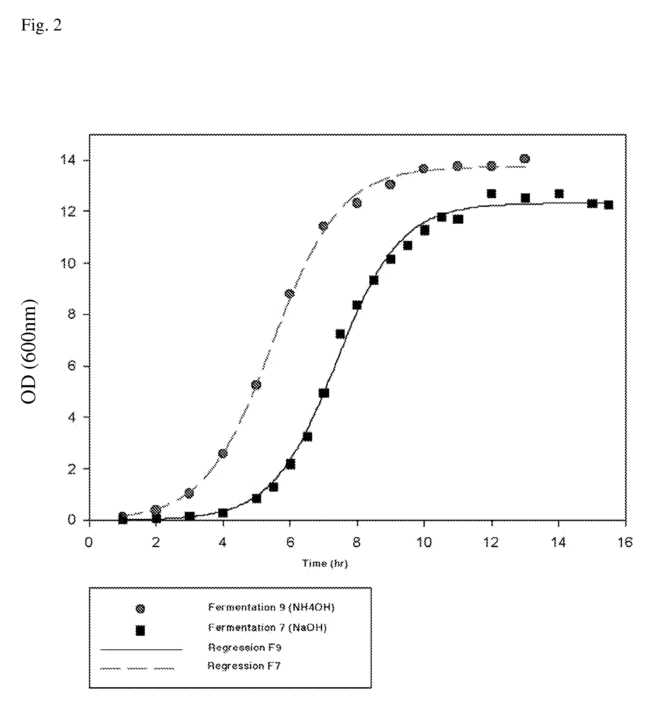

**Figure 2****.** Effect of using NH₄OH or NaOH for adjusting the pH of the culture medium on growth of *Lactococcus Lactis* as determined by measuring OD₆₀₀ values over time.
**Figure 3****.** Monitoring of the glucose concentration in a 501 batch culture process for *Lactococcus Lactis* over time.
**Figure 4****.** Monitoring of growth of *Lactococcus Lactis* bacteria as determined by measuring OD₆₀₀ values in a 501 batch culture process over time.

### Summary of the invention

As outlined hereinbefore, it has been an object of the present invention to provide a culture medium for lactic acid bacteria that does not comprise animal derived compounds and minimally comprises a carbon source, a magnesium source, a nitrogen source, a phosphate buffer and an organic acid.

Accordingly, in a first embodiment, the present invention provides a culture medium for lactic acid bacterial strains characterized by:
- not containing animal derived compounds; and,
- comprising:
   - between and about 10-30 g/l Bacto Yeast Extract;
   - between and about 0.2 - 0.5 g/l MgSO₄.7H₂O;
   - between and about 1-20 g/l K₂HPO₄;
   - between and about 20-75 g/l glucose monohydrate; and
   - about 0,5 g/l of an organic acid.

In a specific embodiment, one or more strains beneficial to the human being can be used as the lactic acid bacterial strains. These may be selected from the group consisting of *Lactococcus genus, Lactobacillus genus, Bifidobacterium* genus, and any variants, mutants and genetically modified bacteria derived thereof.

In a further embodiment of the present invention, the organic acid in the culture medium for the lactic acid bacterial strains is selected from the group of organic acids, in particular citric acid and ascorbic acid.

In a further embodiment, the culture medium of the present invention contains a base for buffering the pH, selected from the group consisting of NaOH, and NH₄OH; in particular a non-sodium containing base such as NH₄OH.

In a preferred embodiment, the present invention provides a culture medium for culturing lactic acid bacterial strains characterized by:
- not containing animal derived compounds; and
- comprising:
   - about 20 g/l Bacto Yeast Extract;
   - about 0,51 g/l MgSO₄.7H₂O;
   - about 2 g/l K₂HPO₄;
   - about 55 g/l glucose monohydrate; and
   - about 0,5 g/l ascorbic acid;

In another preferred embodiment the pH of the culture medium for lactic acid strains is kept constant during culturing of the lactic acid bacterial strains by continuously monitoring and if necessary adjusting the pH by the addition of base selected from NaOH or NH₄OH; preferably NH₄OH.

### Detailed Description of the Invention

A first objective of the invention is to provide an animal component-free culture medium for growing of high numbers of lactic acid bacterial strains to 10⁸ cfu/ml or higher, preferably 10⁹ cfu/ml or higher, more preferably 10¹⁰ cfu/ml or higher

As used herein, the term "lactic acid bacterial strains" discloses gram-positive, microaerophilic or anaerobic bacterial strains that ferment sugars with the production of acids including lactic acid as the predominantly produced acid. The industrially most useful lactic acid bacteria are found among *Lactococcus* spp. *Streptococcus* spp., *Lactobacillus* spp. Additionally, lactic acid bacterial stains belonging to the group of the strictly anaerobic bacteria, i.e. *Bifidobacterium* spp. are generally included in the group of lactic acid bacterial strains. With regard to the present invention, any variants, mutants and genetically modified bacteria derived from the *Lactococcus* spp. *Streptococcus* spp., *Lactobacillus* spp. or *Bifidobacterium* spp are considered to be lactic acid bacterial strains.

The present invention also provides the use of the medium as defined herein for culturing recombinant lactic acid bacterial strains comprising nucleic acids or vectors for expressing heterologous proteins, or for example by introducing exogenous sequences into the thyA locus of Lactococcus (Steidler et al., 2003 Nat .Biotechnol. 21:785-789). Said cultured recombinant lactic acid bacterial strains can subsequently by used for isolation of the recombinant expressed proteins (see, e.g., US 5,559,007), or for delivery, especially therapeutic delivery, of said proteins to subjects (see, e.g., WO 97/14806; WO 01/02570; Steidler et al., 2003).

In this particular embodiment, the culture media of the present invention further comprise a component that should complement a deficiency acquired by the recombinant lactic acid bacteria in order to comply with the requirements of biological containment in a deliberate release of recombinant bacteria in the environment, such as for example, but not limited to, a deficiency in the thymidilate synthase A gene where upon such deficient lactic acid bacteria can only grow when a supplement of thymidine or thymine is present in the culture medium. Culture media containing such supplements are also within the scope of the present invention.

As provided in more detail in the examples hereinafter, the culture medium for said lactic acid bacterial strains does not comprise animal derived compounds and minimally comprises a carbon source, a phosphate buffer, a nitrogen source, a magnesium source and an organic acid.

In one embodiment of the present invention, the culture medium as provided herein, is further **characterized in that** it is substantially free of sodium, i.e. in that, but for the naturally occurring sodium ions present in the nitrogen source, no further sodium ions are added to the culture medium. Accordingly, in a particular embodiment the high-yield animal component-free culture medium for lactic acid bacterial strains comprises a carbon source, a phosphate buffer, a nitrogen source, a magnesium source and an organic acid, **characterized in that** the carbon source, phosphate buffer, magnesium source, and organic acid, are free of sodium.

In principle any non-animal carbon source can be used in the culture medium of the present invention and include for example sugars, alcohols, fats, and aromatic esters, carotenoids and terpenes. Particular examples include the sugars, glucose; maltose or lactose. The concentration of the carbon source used in the culture medium of the present invention depends on the type of lactic acid bacteria and the selected cultivation conditions. In a particular embodiment the carbon source is glucose monohydrate. In a preferred embodiment, the concentration of glucose monohydrate is between and about 20-75 g/l, more particular about 55 g/l.

In principle any phosphate buffer can be used in the culture medium of the present invention, and include for example Na₂HPO₄, NaH₂PO₄, K₂HPO₄, and KH₂PO₄. In a particular embodiment of the present invention the phosphate buffer is free of sodium and preferably selected from K₂HPO₄, KH₂PO₄, or the combination of K₂HPO₄ and KH₂PO₄. Preferably K₂HPO₄ is used as a phosphate buffer at a preferred concentration of between and about 1-20 g/l, more preferably about 1, 2, 3, 4, 5, 6, 7, 8, 9, 10, 11, 12, 13, 14, 15, 16, 17, 18, 19 or about 20 g/l, even more preferably in a concentration range of about 2 - 5 g/l. Alternatively, KH₂PO₄ is used as a phosphate buffer at a preferred concentration of between and about 1-20 g/l, more preferably about 1, 2, 3, 4, 5, 6, 7, 8, 9, 10, 11, 12, 13, 14, 15, 16, 17, 18, 19 or about 20 g/l, even more preferably in a concentration range of about 2 - 5 g/l. When used in combination K₂HPO₄ and KH₂PO₄ are each independently used at a preferred concentration of between and about 1-20 g/l, in particular K₂HPO₄ is present at a concentration of between and about 1-20 g/l and KH₂PO₄ at a concentration of between and about 2 - 5 g/l.

In principle any non-animal magnesium source can be used in the culture medium of the present invention and typically consist of a magnesium salt such as for example MgCl₂, and MgSO₄. In a preferred embodiment the magnesium salt is MgSO₄.7H₂O used at a concentration of between and about 0.1- 2 g/l, preferably about 0.2, 0.3, 0.4, 0.5, 0.6, 0.7, 0.8,0.9, or about 1.0 g/l, even more preferably in a concentration range of about 0.2 - 0.5 g/l.

In principle any non-animal nitrogen source can be used in the culture medium of the present invention and include for example enzymatic digestion or acid hydrolysis of natural products, such as plants or microbial cultures. Particular examples include; enzymatic digests or hydrolysates of plant material like barley, pea, potato, corn and soya; enzymatic digests or hydrolysates of yeast; or enzymatic digests or hydrolysates of protein mixtures including casein. In a particular embodiment of the present invention Bacto Yeast Extract is used as a nitrogen source for the culture medium of the present invention.

The concentration of the nitrogen source used in the culture medium of the present invention depends on the type of lactic acid bacteria and the selected culturing conditions. In a preferred embodiment the nitrogen source is Bacto Yeast Extract , used at a concentration of between and about 5-30 g/l, preferably about 5, 10 or about 20 g/l .

Bacto Yeast Extract as used in the present invention is defined as a water-soluble extract from autolyzed yeast cells suitable for use in cell culture media and microbial fermentation.

Furthermore, any organic acid can be used in the present invention, but preferably ascorbic acid or citric acid is used. In a preferred embodiment the organic acid is used at a concentration of between and about 0.1 - 1 g/l, preferably in a concentration range of about 0.4 - 0.6 g/l.

In a preferred embodiment the pH of the medium of the present invention is kept at between and about 7.0 and 7.5, more preferably to about 7.0 or about 7.5. Adjustment of the pH is preferably obtained by the addition of a base selected from the group consisting of NaOH, and NH₄OH; more preferably by a base not comprising sodium such as NH₄OH.

As used herein the term culture medium is defined as a nutrient medium used in a large-scale fermentation culture wherein microbial cells are grown in a fixed volume under specific environmental conditions (e.g. nutrient type, temperature, pressure, aeration, etc) up to a certain density in a tank or fermentor until they are ready to be harvested and processed as a batch.

A second objective of the invention is to provide a process for culturing lactic acid bacterial strains comprising the use of the culture medium as defined in the present invention.

In a preferred embodiment the process for culturing lactic acid bacterial strains comprises the steps of
- culturing a preculture of the lactic acid bacterial strains in the medium as defined in the present invention to an OD₆₀₀ of not less than about 2.6 or more particularly of 4, 5, 6, 7, 8, 9 or 10
- transferring the preculture to a fermentor containing the culture medium of the present invention
- culturing the bacteria until the glucose concentration has dropped below 0.5 g/l.

The means to evaluate the glucose concentration during the batch culture as used in the present invention are known to the person skilled in the art and comprise methods based on the reducing properties of glucose, such as for example the dinitrosalicylic acid assay (DNS) or the ferricyanide assay; or enzymatic assays such as for example using hexokinase or glucose oxidase.

The time-temperature conditions, pH conditions and aeration conditions during the process for culturing of the bacteria, if relevant, will depend i.e. on the particular type of lactic acid bacterial strain used. Specific examples of such conditions are provided in the following examples. Typically a culturing under standard conditions is run for between and about 8-24 hours, at a rotation speed of between and about 200-1000 rpm at a temperature range of between and about 15-40°C, and at a pH range of between and about 4-8.

In a preferred embodiment the pH is kept constant during the batch cultivation process by continuously monitoring and if necessary adjusting the pH by the addition of a base selected from NaOH or NH₄OH; preferably NH₄OH.

This invention will be better understood by reference to the Experimental Details that follow, but those skilled in the art will readily appreciate that these are only illustrative of the invention as described more fully in the claims that follow thereafter. Additionally, throughout this application, various publications are cited. The disclosure of these publications is hereby incorporated by reference into this application to describe more fully the state of the art to which this invention pertains.

### EXAMPLES

The following examples illustrate the invention. Other embodiments will occur to the person skilled in the art in light of these examples.

The main purpose of the formulation and manufacturing process development was to develop an optimised, non-expensive simple, high-yield animal component-free culture medium.

### EXAMPLE 1: Optimization of OALM medium

To develop an optimized animal-component free culture medium for lactic acid bacterial strains, the frequently used animal-component-containing M 17 medium (table 1) was used as a starting point. Below, the different optimization steps are further explained in more detail with experimental data to support them.

**Table 1: Composition of the M17 medium**

| Component | Concentration |
|---|---|
| Trypton | 5 g/l |
| Soya pepton | 5 g/l |
| Yeast extract | 2,5 g/l |
| Meat extract | 5,0 g/l |
| MgSO₄.7H₂O | 0,51 g/l |
| Na₂ glycerophosphate | 19 g/l |
| Glucose | 50 or 500 g/l |

### Animal-free components and carbon source

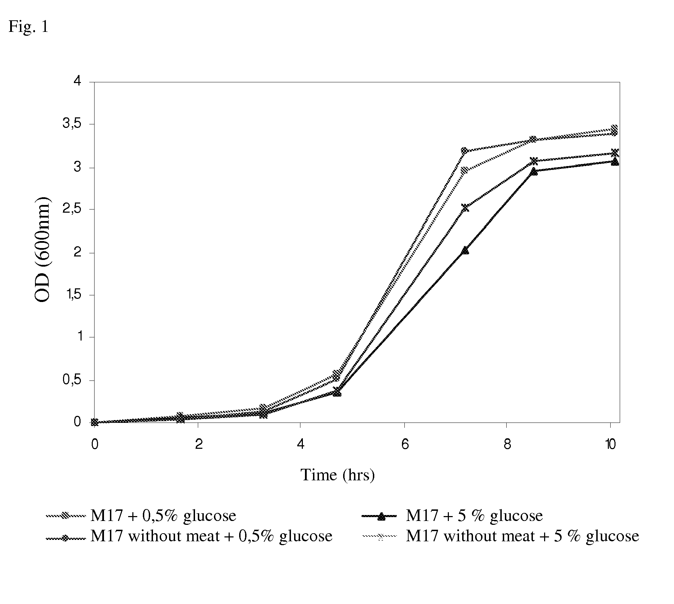

In a first experiment we determined the effect of removing the meat extract from the original M 17 medium on the growth of *Lactococcus Lactis* bacteria. As shown in figure 1, the removal of the meat extract from the M17 medium did not significantly affect the growth of *Lactococcus Lactis* bacteria as determined by the increase in OD₆₀₀ value over time, at 2 concentrations of glucose (5 and 50 g/l).

### Phosphate buffer

As it is known that sodium can cause stress for the bacteria, and the use of Na₂-glycerophosphate as a phosphate source is expensive, we tried and replaced it by K₂HPO₄ in the original M17 medium. Surprisingly, the use of this new phosphate buffer even resulted in a 1,5 times increase in the OD₆₀₀ values.

### Nitrogen source

In the original M17 medium trypton, soya pepton and yeast extract were used as a nitrogen source. Since the optimized medium should be animal-free, multiple animal-free soya peptones alone and/or in combination with Bacto Yeast Extract (BD) were tested for their applicability as a nitrogen source. The tested soya peptones were:
- VG0300 Veggietones (Oxoid)
- VG0100 Veggietones (Oxoid)
- Difco Select Phytone, UF (BD)
- Difco Select Soytone (BD)
- BBL Phytone Peptone (BD)

The optimal ratio of Bacto Yeast Extract and soya pepton was determined by the OD values of bacterial cultures in erlenmeyers with different ratios of Bacto Yeast Extract and the different soya peptones. Since Bacto Yeast Extract alone, always scored better compared to any combination of Bacto Yeast Extract with a soya peptone, it was decided to use only Bacto Yeast Extract at a concentration of 20 g/l for the optimized medium.

### Optimized Actogenix Lactococcus Medium (OALM)

Based on the above-described optimizations to the original M17 medium, the final animal-free medium contains the components as indicated in table 2.

**Table 2: Composition of the OALM medium**

| Component | Concentration |
|---|---|
| Bacto Yeast Extract (BD) | 20 g/l |
| Citric acid monohydrate | 0,49 g/l |
| MgSO₄.7H₂O | 0,51 g/l |
| Glucose | 50 g/l |
| K₂HPO₄ | 2 g/l |

In the aforementioned composition also thymidine has been added to the medium because in this experiment a *Lactococcus lactis* bacterium containing a deficient Thymidilate Synthase A gene has been used for fermentation. Such strain can only grow if thymidine or thymine has been added to the medium as a supplement.

### pH-control and selection of used base

We noticed that keeping the pH, of the OALM medium, steady at 7.0 during culturing of the bacteria resulted in a significant 2,5 times increase in the growth of the bacteria compared to not adjusting the pH during cultivation of the bacteria. These original experiments made use of NaOH as a pH-regulator, however we evaluated whether NH₄OH could be an alternative, since this could provide an extra nitrogen source and as already explained above, sodium can cause stress for the bacteria. Interestingly the use of NH₄OH resulted in an even higher final OD value, indicating that it is indeed a good alternative for NaOH.

### Production process of OALM medium

For the preparation of 501 of OALM medium:
- lkg Bacto Yeast Extract, 25.5 g MgSO₄.7H₂O and 25 g ascorbic acid are dissolved in 51 *aq. dest.* and transferred to the fermentor
- 39.31 *aq. dest* is added to the fermentor and the solution is stirred for 1 hour at 60°C and 500 rpm
- the fermentor containing the solution is than autoclaved for 25 min at 121°C
- 2,750 kg glucose monohydrate and 100 g K₂HPO₄ are dissolved in 51 *aq. dest.* and autoclaved for 1 hour at 121°C
- The glucose-phosphate mixture and thymidine is than added to the fermentor at a temperature below 60°C

### EXAMPLE 2: Optimized batch culture process

Furthermore, based on the optimized OALM medium, a batch culture process was optimized to obtain a maximum yield of *Lactococcus Lactis* bacteria.

The currently optimized culture process is a three step process and includes 2 pre-cultures (in 250 ml and 5 L flasks, filled with 100 ml and 4.9 L of pre-culture medium respectively) and a pH-controlled batch culture step in a 270 L bioreactor (working volume 200 L) with OALM medium.

All pre-culture operations are performed in a Biosafety cabinet. All culture media and buffers are sterilized and are free from materials of animal origin. The in-process controls (IPC) performed on the pre-cultures are:
- identity testing (Gram staining),
- purity testing (bioburden, assessed by plate count),
- optical density testing at 600 nm (OD₆₀₀),
- glucose concentration testing, and
- pH testing.

### Batch culture

The composition of the batch culture medium (AOLM) is depicted in table 2. First, a solution containing Bacto Yeast Extract, Citric acid monohydrate and MgSO₄ is prepared and steam-sterilized *in situ* in the fermentor, after which a 50% glucose + K₂HPO₄ (2 g/l) solution and a 100mM thymidine solution are filtration-sterilized separately and added aseptically. The total medium volume in the fermentor at this point is 175 ± 5L. Next, the sterile culture medium is sampled for bioburden control. The fermentor is subsequently inoculated with the 5 L PC II. Cultivation is performed at 30 °C and a stirrer speed of 200 rpm for approximately 7 to 9 hours. The pH is controlled at 7.0 by addition of sterile 5 M NH₄OH.

The cultivation measurement and control system continuously registers pH, temperature and stirring speed. Samples are taken during the fermentation as IPC for optical density (OD₆₀₀) and glucose concentration. Samples are taken towards the end of the fermentation step to verify culture purity and concentration (CFU/ml). The end of the fermentation process is defined as the time point at which the glucose concentration drops below 0.5 g/l, after which fermentor cooling is initiated.

The IPC for the batch fermentation process consist of:
- purity testing (bioburden, assessed by plate count),
- culture purity testing (Gram staining),
- viable cell count testing (plate count),
- pH testing,
- OD₆₀₀ testing,
- glucose concentration testing.

### Results

During an example batch culture of 200 1 with *Lactococcus Lactis* bacteria, multiple parameters were checked after the end of the culture process which was defined as the timepoint when the glucose was completely consumed by the bacteria (< 0.5 g/L).

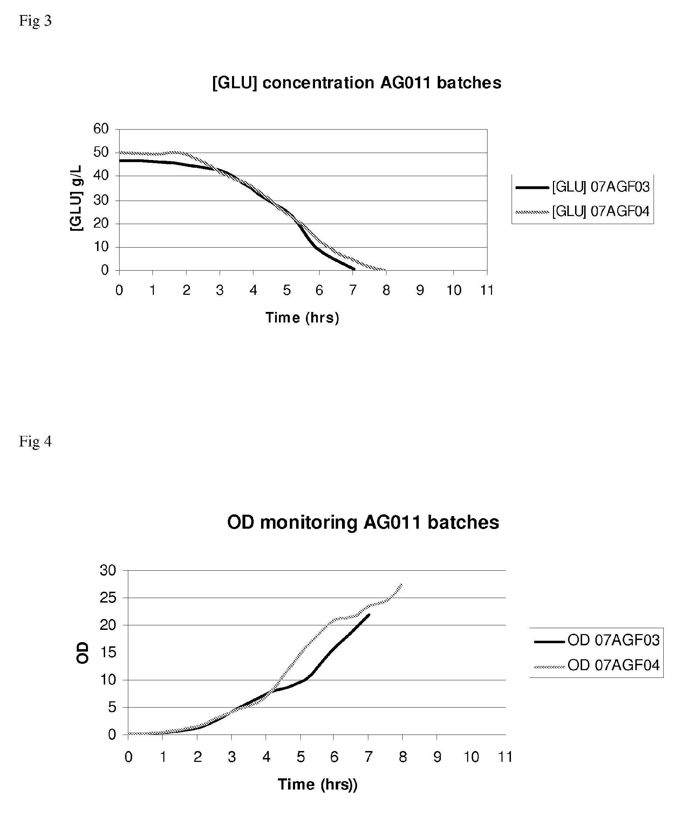

As shown in figure 3, it takes about 7-8 hours before the glucose is completely consumed by the bacteria.

The maximum OD₆₀₀ obtained after these 7-8 hours is 21.96 and 27.5 in 2 different batches, respectively as shown in figure 4.

Furthermore, the number of colony forming units (CFU) (1.2-1.4 x 10¹⁰ CFU/ml) was determined at the end of the culture process by plating multiple dilutions on GM 17 plates supplemented with 0,5% glucose.

The automated addition of a 5 M NH₄OH solution to the fermentor in order to keep the pH at 7.0 was monitored. In total after 7-8 hours of cultivation, 14.8-15.9 kg of the NH₄OH solution was added to the fermentor.

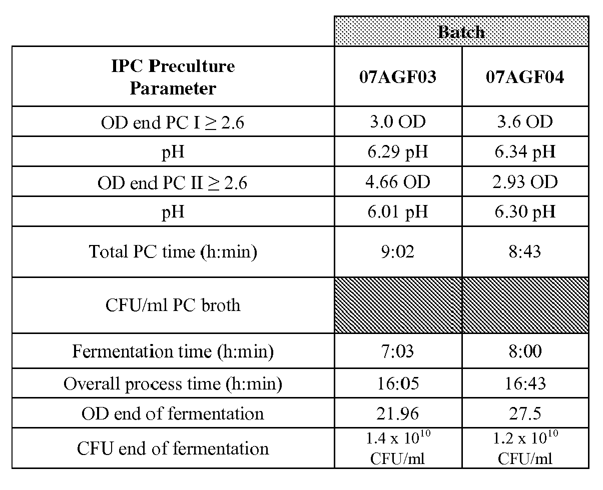

## Claims

1. A culture medium for lactic acid bacterial strains comprising a yeast or plant extract; a phosphate buffer; a magnesium source; a carbon source and an organic acid; and **characterized in that** it does not contain animal derived components,

2. The culture medium according to claim 1 wherein the organic acid is either citric acid or ascorbic acid.

3. The culture medium according to claim 1 or 2 wherein the yeast extract is Bacto Yeast Extract.

4. The culture medium according to any of the claims 1 to 3 wherein the phosphate buffer is K₂HPO₄, KH₂PO_{4.}, or a combination thereof.

5. The culture medium according to any of the claims 1 to 4 wherein the carbon source is glucose, preferably glucose monohydrate.

6. The culture medium according to any of the claims 1 to 5 wherein the magnesium source is MgCl₂ or MgSO₄.

7. A culture medium according to claim 1 comprising about 20 g/l Bacto Yeast Extract; about 0,5 g/l MgSO₄.7H₂O; about 2 g/l K₂HPO₄; about 55 g/l glucose monohydrate and 0,5 g/l ascorbic acid.

8. The culture medium according to claims 1 and 6 for use as a batch culture medium for lactic acid bacterial stains.

9. The culture medium according to any one of claims 1-8 further **characterized in that** it contains a base for buffering the pH, selected from the group consisting of NaOH, and NH₄OH; in particular NH₄OH.

10. The culture medium according to any one of claims 1-9 further **characterized in that** the carbon source, phosphate buffer, magnesium source, and organic acid, are free of sodium.

11. Use of a culture medium according to any one of claims 1-10 for culturing lactic acid bacterial strains.

12. Use of a culture medium according to any one of claims 1-10 for culturing recombinant lactic acid bacterial strains.

13. The use according to any one of claims 11 - 12, wherein the lactic acid bacterial strains are selected from *Lactococcus, Lactobacillus,* and *Bifidobacterium* species.

14. A process for culturing lactic acid bacterial strains as defined in any one of claims 11 - 13, comprising the use of a culture medium as defined in any one of claims 1-10.

15. The process according to claim 14 comprising the steps of
- culturing a pre-culture of said lactic acid bacterial strains in a medium as defined in any one of claims 1-8 until an OD₆₀₀ of 4 or more
- transferring the pre-culture to a fermentor containing the culture medium as defined in any one of claims 1-9
- culturing the bacteria until the glucose concentration has dropped below about 0,5 g/l.

16. The process according to claim 15 wherein the pH is kept constant by continuously monitoring and if necessary adjusting the pH by the addition of base selected from NaOH or NH₄OH; preferably NH₄OH.
